# EUROPÄISCHE PATENTSCHRIFT

(11) **EP 2 825 863 B1**
(45) Veröffentlichungstag und Bekanntmachung des Hinweises auf die Patenterteilung: **19.08.2020**
(21) Anmeldenummer: 13710827.0
(22) Anmeldetag: 12.03.2013
(51) Int. Cl.: G01N 1/22, G01N 33/00

(54) **ANLAGE ZUR ENTNAHME VON ABGASPROBEN VON VERBRENNUNGSKRAFTMASCHINEN**
SYSTEM FOR REMOVING EXHAUST GAS SAMPLES FROM INTERNAL COMBUSTION ENGINES
INSTALLATION DESTINÉE À PRÉLEVER DES ÉCHANTILLONS DE GAZ D'ÉCHAPPEMENT ISSUS DE MOTEURS À COMBUSTION INTERNE.

(30) Priorität: 14.03.2012 DE 102012102137
(43) Veröffentlichungstag der Anmeldung: 21.01.2015
(73) Patentinhaber: AVL Emission Test Systems GmbH, 41460 Neuss (DE)
(72) Erfinder: DICKOW, Achim, 42555 Velbert (DE); BALLIK, Rainer, 47447 Moers (DE); WILLICH, Sascha, 41563 Kaarst (DE)
(74) Vertreter: terpatent Patentanwälte ter Smitten Eberlein-Van Hoof Rütten Daubert
(86) Internationale Anmeldenummer: PCT/EP2013/054973
(87) Internationale Veröffentlichungsnummer: WO 2013/135678

(56) Entgegenhaltungen:
- EP-A1- 0 042 800
- EP-A1- 0 428 850
- WO-A1-2005/045398
- US-A- 5 469 731
- US-A1- 2011 252 864

## Beschreibung

Die Erfindung betrifft eine Anlage zur Entnahme von Abgasproben von Verbrennungskraftmaschinen mit einem Abgaskanal, welcher über einen Abgaseinlass mit einer Abgasquelle fluidisch verbunden ist, einem Luftkanal, in den über einen Luftfilter Umgebungsluft ansaugbar ist, einer Mischzone, die stromabwärts eines Ausströmquerschnitts des Abgaskanals angeordnet ist, einem Verdünnungstunnel, durch den ein Abgas-Luft-Gemisch strömt, wobei ein Ausströmquerschnitt des Abgaskanals im Wesentlichen konzentrisch im Luftkanal angeordnet ist und stromabwärts des Ausströmquerschnitts des Abgaskanals im Verdünnungstunnel eine Ringblende angeordnet ist.

Derartige Anlagen sind beispielsweise unter dem Begriff CVS-Anlage (constant volume sampling) bekannt. Bei diesen Anlagen wird dem Abgas immer so viel Luft beigemischt, dass ein konstanter Gesamtvolumenstrom des Luft-Abgas-Gemisches entsteht. Die über diese Anlagen in Beuteln entnommenen Proben werden im Folgenden bezüglich ihrer Schadstoffanteile analysiert. Insbesondere werden der Kohlendioxid-, der Kohlenmonoxid-, Kohlenwasserstoff-, der Stickoxid-Anteil sowie die Partikelbelastung gemessen. Die Art der Messungen ist gesetzlich beispielsweise durch die ECE-Richtlinie R 83 für den europäischen Raum oder den Code of federal regulations Gesetz Nr.40 für den US-amerikanischen Raum geregelt.

So wird in der DE 10 2009 015 188 A1 eine Anlage zur Entnahme von Abgasproben beschrieben, die an zwei verschiedene Abgasquellen angeschlossen werden kann, so dass über eine Anlage sowohl die Partikelbelastung von Dieselmotoren als auch von Ottomotoren gemessen werden kann. Der Abgaskanal der Anlage, der vom Dieselmotor kommt, endet konzentrisch im Luftkanal stromaufwärts einer Blende. Im Bereich zwischen dem Austrittsquerschnitt des Abgaskanals und der Blende bildet sich eine Mischzone, in der das Abgas sich mit der Luft mischt.

Es hat sich jedoch gezeigt, dass in einem derartigen System Strömungssträhnen entstehen können, in denen die Abgasdurchmischung schlechter ist als in anderen Bereichen. Eine gleichmäßige Durchmischung ist somit trotz der relativ langen Laufstrecken nicht gegeben, so dass die Messergebnisse verfälscht werden können.

In der EP 0 428 850 A1 wird ein Verdünnungssystem beschrieben, bei dem ein Abgasteilstrom aus einer Abgasquelle in den zunächst von Luft durchströmten Tell eines Verdünnungstunnels einströmt. In den Verdünnungstunnel ragen stromabwärts der Abgaseinleitung mehrere symmetrisch angeordnete Teilungsverhältnis-Steuerdüsen. Diese Düsen sind mit einer Druckluftquelle verbunden. Entsprechend kann, falls gewünscht, zusätzliche Luft über die Düsen in den Verdünnungstunnel eingebracht werden. Diese zusätzliche Lufteinleitung dient dazu, dass der statische Druck am Auslass des Abgaseinleitrohres gleich dem statischen Druck am Mehrrohrströmungsverteiler im Abgassystem ist.

Daher stellt sich die Aufgabe, eine Anlage zu entwickeln, mit welcher entsprechend der gesetzlichen Vorschriften eine repräsentative Probenentnahme sichergestellt wird, indem die Bildung von Gebieten mit schlechter Abgasdurchmischung vermieden wird. Gleichzeitig soll die Anlage kostengünstig herstellbar und betreibbar sein, insbesondere sollen Förderpumpen mit relativ kleiner Leistung zur Förderung der beiden Gasströme ausreichen.

Diese Aufgabe wird durch eine Anlage zur Entnahme von Abgasproben von Verbrennungskraftmaschinen mit den Merkmalen des Hauptanspruchs gelöst. Dadurch, dass stromaufwärts der Blende und stromabwärts des Ausströmquerschnitts des Abgaskanals Mittel angeordnet sind, über welche der Abgasstrom derart abgelenkt wird, dass sich der Abgasstrom unmittelbar stromabwärts der Mittel achssymmetrisch von der Mittelachse des Verdünnungstunnels entfernt, wird das Abgas direkt in den Luftstrom geleitet, der zuvor aufgrund des in den Luftkanal ragenden Abgaskanals in den äußeren Bereich des Verdünnungstunnels verdrängt wurde, wodurch die Durchmischung deutlich erhöht wird. Durch die Blende erhöht sich anschließend die Geschwindigkeit des Abgas-Luft-Gemisches, wodurch zusätzliche Turbulenzen gebildet werden, was zu einer zusätzlichen Homogenisierung des Mischstroms führt. Dabei sind die Mittel zur Strömungsablenkung entweder durch einen Körper gebildet, der eine Kugelzone aufweist und konzentrisch im Verdünnungstunnel angeordnet ist oder durch mehrere zur Mittelachse symmetrisch zueinander angeordnete Ausströmrohre. Durch die Kugelzone wird der Druckverlust bei der Ablenkung der Abgasströmung nicht wesentlich erhöht, so dass das treibende Druckgefälle und somit die Leistungsaufnahme einer Förderpumpe nicht erhöht werden müssen. Auch wurden mit diesem Körper besonders gute Durchmischungen der beiden Gasströme erreicht. Bei der Ausführung, bei der die Mittel zur Strömungsablenkung durch mehrere zur Mittelachse symmetrisch zueinander angeordnete Ausströmrohre gebildet sind, wird ebenfalls Druckverlust reduziert, da kein Strömungshindernis hinter dem Abgaskanal angeordnet Ist als die Blende. Dennoch wird der Abgasstrom in Richtung des Luftstroms umgeleitet.

In einer hierzu weiterführenden Ausführungsform ist die Kugelzone eine Halbkugel. Diese Ausführung brachte besonders gute Ergebnisse bei der Homogenisierung der Mischung und gleichzeitiger Reduzierung des Druckverlustes.

Um Partikelablagerungen zu verhindern, ist die Oberfläche der Kugelzone elektropoliert. Des Weiteren wird verhindert, dass sich später von den Ablenkungsmitteln lösende Partikel das Messergebnis verfälschen.

In einer vorteilhaften Ausgestaltung der Kugelzone weist diese ein zentrales Loch auf. Durch dieses Loch wird die Ausbildung eines den Druckverlust deutlich erhöhenden Staupunktes vermieden, ohne die Durchmischung deutlich zu verschlechtern.

Weiterhin sind vorzugsweise die Kugelzone oder die Blende beheizbar. Dies wird besonders einfach verwirklicht, indem auf der stromabwärtigen Oberfläche der Kugelzone oder der Blende eine Heizfolie angeordnet ist, so dass diese nicht direkt dem Abgasstrom ausgesetzt wird, sondern durch die stromaufwärtige Oberfläche geschützt wird. So wird eine Kondensatbildung auf den angeströmten Oberflächen vermieden, was wiederum zu einer Reduzierung der Ablagerungen führt.

Besonders gute Ergebnisse werden erzielt, wenn die Projektionsfläche der Kugelzone in Richtung der Mittelachse zumindest dem Ausströmquerschnitt des Abgaskanals entspricht, da einerseits sichergestellt wird, dass der gesamte Abgasstrom in Richtung des Luftstroms umgelenkt wird und andererseits eine zusätzliche Einschnürung, die zu einem Druckverlust führen würde, verhindert wird.

In einer weiteren bevorzugten Ausbildung der Erfindung entspricht der Durchströmungsquerschnitt der Blende dem 1,2 bis 1,8-fachen des Querschnitts der Mittel zur Strömungsablenkung. So wird die Durchströmungsgeschwindigkeit erhöht und es werden Turbulenzen erzeugt, die die Durchmischung weiter verstärken, jedoch ohne dass zu große Druckverluste die Folge wären.

In einer weiterführenden Ausführung beträgt der Abstand der Mittel zur Strömungsablenkung vom Ausströmquerschnitt des Abgaskanals ein Drittel bis ein Fünftel der Differenz des Durchmessers des Luftkanals und des Durchmessers des Abgaskanals. So wird sichergestellt, dass der Druckverlust beim Einströmen des Abgases in den Luftstrom nicht zu groß wird. Einerseits wird dem Abgasstrom ausreichend Ausströmfläche zur Verfügung gestellt und andererseits ist der Ablenkwinkel ausreichend hoch um Turbulenzen zu erzeugen und so eine Strähnenbildung verhindern zu können.

Ein zu großer Druckverlust beim Durchströmen der Blende wird dadurch verhindert, dass der Abstand der Mittel zur Strömungsablenkung zur Blende ein Drittel bis ein Fünftel des Durchmessers des Luftkanals beträgt. So wird einerseits eine ausreichende Fläche für das Abgas-Luftgemisch zur Verfügung gestellt und andererseits durch die relativ nah aufeinanderfolgende Umlenkung in entgegengesetzte Richtungen eine Turbulenzbildung erzeugt.

Bevorzugt sind vier Ausströmrohre um 90° versetzt zueinander angeordnet. So entsteht eine ausreichend konstante Einleitung des Abgases über den gesamten Querschnitt, jedoch mit geringem konstruktiven Aufwand. Auch ist diese Ausführung leicht zu montieren.

In einer Weiterführung dieser Erfindung erstrecken sich die vier Ausströmrohre in unmittelbarer Nähe des Abgaskanals oder im Abgaskanal zunächst parallel zur Mittelachse und sind im folgenden Verlauf außerhalb des Abgaskanals stetig von der Mittelachse entfernend gebogen. Durch diese stetige Biegung werden die Druckverluste reduziert.

In einer vorteilhaften Ausgestaltung schließen die Austrittsquerschnitte der Ausströmrohre zur Mittelachse des Verdünnungstunnels einen Winkel von 30° bis 80° ein, wodurch einerseits eine ausreichende Ablenkung zur Erhöhung der Durchmischung sichergestellt wird und andererseits der Druckverlust beim Aufeinandertreffen der beiden Gasströme in vertretbaren Grenzen gehalten wird, so dass die Gesamtdruckerhöhung der Gebläse zur Förderung des Gasstroms nicht weiter angepasst werden müssen.

Eine besonders einfache Befestigung und Montage ergibt sich, wenn die Mittel zur Strömungsablenkung über zumindest einen, vorzugsweise drei Halter an der Blende befestigt sind, da die Montage in einem Schritt mit der Blendenmontage erfolgt.

Es wird somit eine Anlage zur Entnahme von Abgasproben von Verbrennungskraftmaschinen geschaffen, mit der sichergestellt wird, dass repräsentative Proben aus dem Verdünnungstunnel genommen werden können, da eine sehr gute Durchmischung der beiden Gasströme sichergestellt wird. Andererseits ist die Anlage kostengünstig herstellbar und betreibbar, da die Montage einfach ist und relativ kleine Fördergebläse verwendet werden können, da zusätzliche Druckverluste klein gehalten werden.

Zwei Ausführungsbeispiele einer erfindungsgemäßen Anlage zur Entnahme von Abgasproben sind in den Figuren dargestellt und werden nachfolgend beschrieben.
Figur 1 zeigt schematisch eine erfindungsgemaße Anlage zur Entnahme von Abgasproben in Seitenansicht.
Figur 2 zeigt in vergrößerter Darstellung einen Ausschnitt der Anlage aus Figur 1 im Bereich der Mischzone in dreidimensionaler Darstellung.
Figur 3 zeigt eine alternative erfindungsgemäße Ausbildung der Mischzone in dreidimensionaler Darstellung.

Die erfindungsgemäße Anlage zur Entnahme von Abgasproben von Verbrennungskraftmaschinen für Diesel- oder Ottomotoren besteht aus einem Abgaseinlass 10, über den ein Abgaskanal 12 mit einer Abgasquelle 14 fluidisch verbunden ist, die durch einen Verbrennungsmotor eines Kraftfahrzeugs gebildet ist.

Dieser Abgaskanal 12 weist ein Ende 16 mit einem Ausströmquerschnitt 18 auf, welches konzentrisch innerhalb eines Luftkanals 20 mündet. Dazu weist dieser Luftkanal 20 eine Öffnung 22 in seiner Begrenzungswand 24 auf, durch die der Abgaskanal 12 senkrecht in den Luftkanal 20 ragt. Um konzentrisch im Luftkanal 20 zu münden, weist der Abgaskanal 12 eine 90°-Umlenkung auf.

Stromaufwärts des Abgaskanals 12 weist der Luftkanal 20 einen Einlass 26 auf, an dem ein üblicherweise aus drei Filtern bestehender, erster Luftfilter 28 angeordnet ist, über den Luft in den Luftkanal 20 eingesaugt werden kann. Die Umlenkung des Abgaskanals 12 ist so ausgeführt, dass die Ausströmrichtung des Abgases zur zum Luftfilter 28 entgegengesetzten Seite gerichtet ist, so dass die Luftströmung und die Abgasströmung am Ausströmquerschnitt 18 des Abgaskanals 12 eine gemeinsame Strömungsrichtung aufweisen, die im Wesentlichen parallel zur Mittelachse des Luftkanals 20 verläuft.

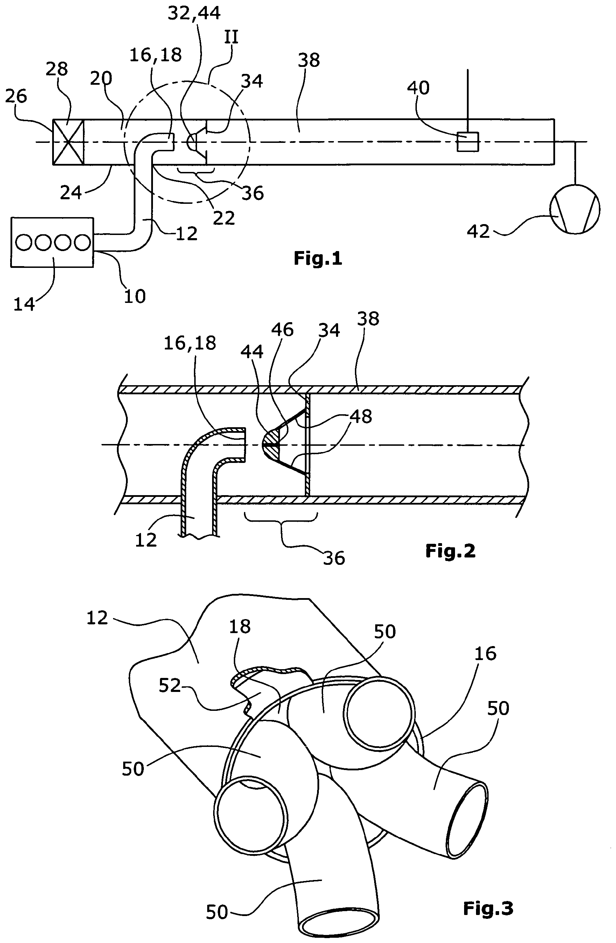

In Strömungsrichtung sind hinter dem Ausströmquerschnitt 18 des Abgaskanals 12 Mittel 32 zur Ablenkung der Abgasströmung angeordnet. Diese können konstruktiv unterschiedlich ausgebildet werden, wie aus der folgenden Beschreibung der Figuren 2 und 3 hervorgeht. Unabhängig von der Ausführung der Mittel 32 dienen diese dazu, den Abgasstrom in Richtung des Luftstroms, also mit einer Komponente in radialer Richtung des Luftkanals 20 in den Luftstrom abzulenken, wodurch Turbulenzen entstehen und eine Verbesserung der Durchmischung auftritt.

Hinter diesen Mitteln 32 zur Strömungsablenkung ist eine Blende 34 angeordnet, durch welche der frei durchströmbare Querschnitt des Luftkanals verengt wird, so dass sich die Strömungsgeschwindigkeit erhöht und zusätzliche Turbulenzen entstehen. Diese Turbulenzzonen bilden eine Mischzone 36, in der eine möglichst vollständige Vermischung des Abgasstroms im Luftstrom erfolgt und die sich bis hinter die Blende 34 zieht.

An diese Mischzone 36 schließt sich ein Verdünnungstunnel 38 an, in dem ein gleichmäßiger Strom des Abgas-Luft-Gemisches ohne Strähnenbildung vorhanden ist. Im Verdünnungstunnel 38 ist zentral zur Mittelachse eine Probenahmesonde 40 zur Entnahme einer Probe aus dem Mischstrom angeordnet. Der über die Probenahmesonde 40 aufgenommene Probestrom kann entweder über einen beheizbaren Partikelfilter einem Flammenionisationsdetektor zugeführt werden, über den die Kohlenwasserstoffe im Abgas bestimmt werden, um die Anteile an Stickoxiden, Kohlendioxid und Kohlenmonoxid im Abgas zu ermitteln. Über die Probenahmesonde 40 werden die Partikelemissionen entnommen und einer Partikelmesseinrichtung zugeführt. Die Förderung der Analyseströme erfolgt jeweils über nicht dargestellte separate Pumpen.

Der übrige Mischgasstrom gelangt vom Verdünnungstunnel 38 zu einer regelbaren Förderpumpe 42, die zur Erzeugung eines ausreichenden Druckes zur Förderung der Luft und des Abgases bestimmt ist. Hier wird der Mischgasstrom ausgestoßen. Neben einer geregelten Förderpumpe 42 ist es ebenfalls möglich, vor der Förderpumpe eine Venturidüse zur Einstellung des gewünschten Förderstroms anzuordnen.

Es wird noch darauf hingewiesen, dass der Gasdurchfluss durch die Förderpumpe bei konstanter Temperatur und konstantem Druck beziehungsweise konstantem Volumenstrom gemessen wird. In jedem Fall wird das Abgas in einem definierten Verhältnis mit der Umgebungsluft verdünnt. Die Probenahme erfolgt jeweils proportional zum Durchfluss der Förderpumpe 42. Hierzu sind Probenahmesysteme mit variabler Verdünnung und Verdrängerpumpe ebenso bekannt wie Verdünnungssysteme mit kritisch durchströmten Venturirohr, wie sie beispielsweise in der ECE Richtlinie R 83 beschrieben werden. Die Anordnung der in diesen Anlagen verwendeten Durchflussreglern, Ventilen, Durchfluss-, Druck- und Temperaturmessgeräten ist ebenfalls bekannt und je nach verwendetem System unterschiedlich, so dass diese Möglichkeiten der Regelung hier als Fachwissen vorausgesetzt werden. Die vorliegende Erfindung ist für alle diese Formen der Probenahme geeignet.

Die Ergebnisse der Probenahme einer solchen Anlage sind jedoch nur dann tatsächlich repräsentativ, wenn sichergestellt wird, dass eine möglichst homogene Strömung an den Probenentnahmestellen vorhanden ist, also eine gute Durchmischung des Abgasstroms im Luftstrom sichergestellt wird und eine Strähnenbildung zuverlässig vermieden wird. Wie bereits ausgeführt erfolgt dies erfindungsgemäß mit Hilfe der Mittel 32 zur Strömungsablenkung. Diese werden in der Ausführung gemäß der Figur 2 durch einen Körper gebildet der Kugelzonenförmig ist, im vorliegenden Fall eine Halbkugel 44 bildet, deren konvexe Oberfläche zum Abgaskanal 12 hin gerichtet ist.

Diese Halbkugel 44 befindet sich in einem Abstand von 45 mm zum Ausströmquerschnitt 18 des Abgaskanals 12 und weist einen Durchmesser von ca. 95 mm auf, während der Ausströmquerschnitt des Abgaskanals 12 etwa 90 mm beträgt. Die Halbkugel 44 wiederum ist etwa 70 mm von der Blende 34 entfernt, deren freier Durchströmungsdurchmesser etwa 150 mm beträgt. Diese Abmessungen wurden in Abhängigkeit des Durchmessers des Luftkanals 20 festgelegt, der im vorliegenden Ausführungsbeispiel 273 mm beträgt, so dass der Durchströmungsquerschnitt der Blende 34 etwa dem 1,6-fachen des Querschnitts der Mittel 32 zur Strömungsablenkung entspricht, der Abstand der Mittel 32 zur Strömungsablenkung vom Ausströmquerschnitt 18 des Abgaskanals 12 etwa ein Viertel der Differenz des Durchmessers des Luftkanals 20 und des Durchmessers des Abgaskanals 12 beträgt und der Abstand der Mittel 32 zur Strömungsablenkung zur Blende 34 etwa ein Viertel des Durchmessers des Luftkanals 20 beträgt.

Diese genannten Verhältnisse sorgen einerseits dafür, dass die zur Verfügung stehende freie Zylinderfläche zum Ausströmen des Abgases aus dem Zwischenraum zwischen dem Abgaskanal 12 und der Halbkugel 44 größer ist als die Ausströmfläche aus dem Abgaskanal 12, wodurch der Druckverlust gering gehalten wird, andererseits jedoch auch dafür, dass der Abstand klein genug bleibt, um eine deutliche Strömungsablenkung nach radial außen an der Halbkugel 44 zu erzeugen, so dass der Abgasstrom in den Luftstrom gedrückt wird, wodurch Turbulenzen entstehen. Des Weiteren wird auch der zur Verfügung stehende Raum zur Durchströmung für das Abgas-Luftgemisch ausreichend groß gehalten, indem die Zylinderfläche zwischen der Blende 34 und der Halbkugel 44 etwa so groß gehalten wird wie der freie Durchströmungsquerschnitt parallel zur Mittelachse in Höhe der Halbkugel 44, so dass auch hier keine zu hohen Druckverluste entstehen. Die ohnehin noch Wirbel behaftete Strömung des Abgas-Luftgemisches wird in diesem Bereich durch die Blende 34 wieder nach radial innen gerichtet, wodurch Wirbel in entgegengesetzter Richtung entstehen, was endgültig zu Turbulenzen führt, die eine sehr gute Durchmischung sicherstellen. Diese Turbulenzen bleiben noch in einem gewissen Abstand hinter der Blende aufrecht erhalten. Dieser Bereich, in dem das Abgas in die Luft einströmt und sich mit dieser mischt, bildet die Mischzone 36.

Die Halbkugel 44 weist des Weiteren ein zentrales Loch 46 auf, welches das Entstehen eines Staupunktes verhindert und somit ebenfalls einen weiteren Druckverlust vermeidet. Die Oberfläche der Halbkugel 44 ist auf ihrer zur Strömung gerichteten Seite elektropoliert auf ihrer entgegengesetzten Seite mit einer Heizfolie beschichtet, so dass Kondensieren von Abgas vermieden wird. Die Halbkugel 44 ist über drei Streben 48 an der Blende 34 befestigt, so dass die Halbkugel 44 auf einfache Weise mit der Blende 34 vormontiert und gemeinsam mit der Blende 34 konzentrisch zum Verdünnungstunnel 38 in diesen eingesetzt werden kann. Durch diese Mittel 32 und deren Anordnung im Kanal 20 wird sichergestellt, dass eine zur Mittelachse symmetrische Strömungsablenkung des Abgases erfolgt, was eine gleichmäßige Durchmischung mit der Luft sicherstellt.

Eine alternative Ausführung zeigt die Figur 3. Im Ausströmquerschnitt 18 des Abgaskanals 12 befinden sich vier um 90° versetzt symmetrisch zueinander angeordnete Ausströmrohre 50, die zunächst parallel zur Durchströmungsrichtung des Abgaskanals 12 verlaufen und hinter dem Ende 16 des Abgaskanals 12 nach radial außen gebogen sind, so dass sich die Rohre 50 in ihrem Verlauf in Strömungsrichtung stetig von der Mittelachse entfernen. Die Austrittsquerschnitte der Ausströmrohre 50 schließen dabei zur Mittelachse des Verdünnungstunnels 38 einen Winkel von 45° ein. Die Befestigung dieser Ausströmrohre 50 erfolgt vorab an einem Ring 52 der bei der Montage am Abgaskanal 12 befestigt wird. Alternativ ist auch eine Befestigung über Streben an der Blende 34 durchführbar.

Auch diese Ausführung der Mittel 32 zur Strömungsablenkung sorgt für ein achssymmetrisches Einströmen des Abgasstroms unter einem Winkel in den Luftstrom, so dass dem Abgasstrom eine radiale Komponente aufgezwungen wird, die dazu führt, dass Wirbel in einer Richtung entstehen, die den Wirbeln aufgrund der Einschnürung durch die Blende 34 entgegengesetzt ist, so dass große Turbulenzen entstehen, die eine sehr gute Durchmischung der beiden Gasströme zur Folge haben. Die stetige Umlenkung sorgt für geringe Druckverluste.

Beide Ausführungen eignen sich entsprechend zur Verbesserung der Durchmischung und Homogenisierung des Abgasstroms, so dass eine repräsentative Probeentnahme ermöglicht wird. Dies führt zu besseren Versuchsergebnissen bei der Analyse der Schadstoffbelastungen, ohne dass die Leistung der Förderpumpen erhöht werden muss, da vorhandene Druckverluste möglichst klein gehalten werden.

Es sollte deutlich sein, dass die vorliegende Erfindung nicht auf das beschriebene Ausführungsbeispiel beschränkt ist, sondern verschiedene Modifikationen innerhalb des Schutzbereichs des vorliegenden Hauptanspruchs möglich sind.

## Patentansprüche

1. Anlage zur Entnahme von Abgasproben von Verbrennungskraftmaschinen mit
einem Abgaskanal (12), welcher über einen Abgaseinlass (10) mit einer Abgasquelle (14) fluidisch verbunden ist,
einem Luftkanal (20), In den über einen Luftfilter (28) Umgebungsluft ansaugbar ist,
einer Mischzone (36), die stromabwärts eines Ausströmquerschnitts (18) des Abgaskanals (12) angeordnet ist,
einem Verdünnungstunnel (38), durch den ein Abgas-Luft-Gemisch strömt,
wobei der Ausströmquerschnitt (18) des Abgaskanals (12) im Wesentlichen konzentrisch im Luftkanal (20) angeordnet ist und stromabwärts des Ausströmquerschnitts (18) des Abgaskanals (12) im Verdünnungstunnel (38) eine Ringblende (34) angeordnet ist,
**dadurch gekennzeichnet, dass**
stromaufwärts der Blende (34) und stromabwärts des Ausströmquerschnitts (18) des Abgaskanals (12) Mittel (32, 44, 50) angeordnet sind, die durch mehrere zur Mittelachse symmetrisch zueinander angeordnete Ausströmrohre (50) oder durch einen Körper (44) der eine Kugelzone aufweist und konzentrisch im Verdünnungstunnel (38) angeordnet ist, gebildet sind, wobei über die Mittel (32, 44, 50) der Abgasstrom derart abgelenkt wird, dass sich der Abgasstrom unmittelbar stromabwärts der Mittel (32, 44, 50) achssymmetrisch von der Mittelachse des Verdünnungstunnels (38) entfernt.

2. Anlage zur Entnahme von Abgasproben von verbrennungskraftmaschinen nach Anspruch 1,
**dadurch gekennzeichnet, dass**
die Kugelzone eine Halbkugel (44) ist.

3. Anlage zur Entnahme von Abgasproben von Verbrennungskraftmaschinen nach einem der vorhergehenden Ansprüche,
**dadurch gekennzeichnet, dass**
die Kugelzone (44) elektropoliert ist.

4. Anlage zur Entnahme von Abgasproben von Verbrennungskraftmaschinen nach einem der vorhergehenden Ansprüche,
**dadurch gekennzeichnet, dass**
die Kugelzone (44) ein zentrales Loch (46) aufweist.

5. Anlage zur Entnahme von Abgasproben von Verbrennungskraftmaschinen nach einem der vorhergehenden Ansprüche,
**dadurch gekennzeichnet, dass**
die Kugelzone (44) oder die Blende (34) beheizbar sind.

6. Anlage zur Entnahme von Abgasproben von Verbrennungskraftmaschinen nach Anspruch 5,
**dadurch gekennzeichnet, dass**
auf der stromabwärtigen Oberfläche der Kugelzone (44) oder der Blende (34) eine Heizfolie angeordnet ist.

7. Anlage zur Entnahme von Abgasproben von Verbrennungskraftmaschinen nach einem der vorhergehenden Ansprüche,
**dadurch gekennzeichnet, dass**
die Projektionsfläche der Kugelzone(44) in Richtung der Mittelachse zumindest dem Ausströmquerschnitt (18) des Abgaskanals (12) entspricht.

8. Anlage zur Entnahme von Abgasproben von Verbrennungskraftmaschinen nach einem der vorhergehenden Ansprüche,
**dadurch gekennzeichnet, dass**
der Durchströmungsquerschnitt der Blende (34) dem 1,2 bis 1,8-fachen des Querschnitts der Mittel (32, 44) zur Strömungsablenkung entspricht.

9. Anlage zur Entnahme von Abgasproben von Verbrennungskraftmaschinen nach einem der vorhergehenden Ansprüche,
**dadurch gekennzeichnete dass**
der Abstand der Mittel (32, 44) zur Strömungsablenkung vom Ausströmquerschnitt (18) des Abgaskanals (12) ein Drittel bis ein Fünftel der Differenz des Durchmessers des Luftkanals (20) und des Durchmessers des Abgaskanals (12) beträgt.

10. Anlage zur Entnahme von Abgasproben von Verbrennungskraftmaschinen nach einem der vorhergehenden Ansprüche,
**dadurch gekennzeichnet, dass**
der Abstand der Mittel (32, 44, 50) zur Strömungsablenkung zur Blende (34) ein Drittel bis ein Fünftel des Durchmessers des Luftkanals (20) beträgt.

11. Anlage zur Entnahme von Abgasproben von Verbrennungskraftmaschinen nach Anspruch 1,
**dadurch gekennzeichnet, dass**
vier Ausströmrohre (50) um 90° versetzt zueinander angeordnet sind.

12. Anlage zur Entnahme von Abgasproben von Verbrennungskraftmaschinen nach Anspruch 11,
**dadurch gekennzeichnet, dass**
die vier Ausströmrohre (50) sich in unmittelbarer Nähe des Abgaskanals (12) oder im Abgaskanal (12) zunächst parallel zur Mittelachse erstrecken und im folgenden Verlauf außerhalb des Abgaskanals (12) stetig von der Mittelachse entfernend gebogen sind.

13. Anlage zur Entnahme von Abgasproben von Verbrennungskraftmaschinen nach Anspruch 12,
**dadurch gekennzeichnet, dass**
die Austrittsquerschnitte der Ausströmrohre (50) zur Mittelachse des Verdünnungstunnels einen Winkel von 30° bis 80° einschließen.

14. Anlage zur Entnahme von Abgasproben von Verbrennungskraftmaschinen nach einem der vorhergehenden Ansprüche,
**dadurch gekennzeichnet, dass**
die Mittel (32, 44, 50) zur Strömungsablenkung über zumindest einen, vorzugsweise drei Streben (48) an der Blende (34) befestigt sind.

## Claims

1. A system for taking exhaust gas samples from internal combustion engines comprising
an exhaust gas duct (12) which is in fluid communication with an exhaust gas source (14) via an exhaust gas inlet (10),
an air duct (20) into which ambient air is adapted to be taken in via an air filter (28),
a mixing zone (36) which is arranged downstream of an outflow cross section (18) of said exhaust gas duct (12),
a dilution tunnel (38) through which an exhaust gas/air mixture flows, wherein said outflow cross section (18) of said exhaust gas duct (12) is essentially concentrically arranged in said air duct (20), and
downstream of said outflow cross section (18) of said exhaust gas duct (12) in said dilution tunnel (38) an annular orifice (34) is arranged,
**characterized in that**
upstream of said orifice (34) and downstream of said outflow cross section (18) of said exhaust gas duct (12) means (32, 44, 50) are arranged that are defined by a plurality of outflow pipes (50) arranged symmetrically to each other relative to the center axis or are defined by a body (44) which comprises a spherical zone and is concentrically arranged in the dilution tunnel (38), wherein, via said means (32, 44, 50), the exhaust gas flow is deflected such that the exhaust gas flow departs in an axially symmetrical manner from the center axis of said dilution tunnel (38) immediately downstream of said means (32, 44, 50).

2. The system for taking exhaust gas samples from internal combustion engines according to claim 1,
**characterized in that**
the spherical zone is a hemisphere (44).

3. The system for taking exhaust gas samples from internal combustion engines according to any one of the preceding claims,
**characterized in that**
the spherical zone (44) is electropolished.

4. The system for taking exhaust gas samples from internal combustion engines according to any one of the preceding claims,
**characterized in that**
the spherical zone (44) comprises a central hole (46).

5. The system for taking exhaust gas samples from internal combustion engines according to any one of the preceding claims,
**characterized in that**
the spherical zone (44) or the orifice (34) is adapted to be heated.

6. The system for taking exhaust gas samples from internal combustion engines according to claim 5,
**characterized in that**
on the downstream surface of the spherical zone (44) or the orifice (34) a heating film is arranged.

7. The system for taking exhaust gas samples from internal combustion engines according to any one of the preceding claims,
**characterized in that**
the projection surface of the spherical zone (44) in the direction of the center axis corresponds at least to the outflow cross section (18) of the exhaust gas duct (12).

8. The system for taking exhaust gas samples from internal combustion engines according to any one of the preceding claims,
**characterized in that**
the flow cross section of the orifice (34) corresponds to 1.2 to 1.8 times the cross section of the means (32, 44) for flow deflection.

9. The system for taking exhaust gas samples from internal combustion engines according to any one of the preceding claims,
**characterized in that**
the distance of the means (32, 44) for flow deflection to the outflow cross section (18) of the exhaust gas duct (12) is one third to one fifth of the difference between the diameter of the air duct (20) and the diameter of the exhaust gas duct (12).

10. The system for taking exhaust gas samples from internal combustion engines according to any one of the preceding claims,
**characterized in that**
the distance of the means (32, 44, 50) for flow deflection to the orifice (34) is one third to one fifth of the diameter of the air duct (20).

11. The system for taking exhaust gas samples from internal combustion engines according to claim 1,
**characterized in that**
four outflow pipes (50) are arranged offset from each other by 90°.

12. The system for taking exhaust gas samples from internal combustion engines according to any one of claims 11,
**characterized in that**
the four outflow pipes (50) in the immediate vicinity of the exhaust gas duct (12) or in the exhaust gas duct (12) at first extend in parallel to the center axis, and in the further course outside said exhaust gas duct (12) are continuously bent away from the center axis.

13. The system for taking exhaust gas samples from internal combustion engines according to claim 12,
**characterized in that**
the discharge cross sections of the outflow pipes (50) include an angle of 30° to 80° to the center axis of the dilution tunnel.

14. The system for taking exhaust gas samples from internal combustion engines according to any one of the preceding claims,
**characterized in that**
the means (32, 44, 50) for flow deflection are fastened to the orifice (34) via at least one, preferably three braces (48).

## Revendications

1. Système de prélèvement d'échantillons de gaz d'échappement à partir de moteurs à combustion interne comprenant
un conduit de gaz d'échappement (12) raccordé de manière fluidique à une source de gaz d'échappement (14) via une entrée de gaz d'échappement (10),
un conduit d'air (20) dans lequel de l'air ambiant peut être aspiré via un filtre à air (28),
une zone de mélange (36) disposée en aval d'une section transversale de sortie (18) du conduit de gaz d'échappement (12),
un tunnel de dilution (38) à travers lequel s'écoule un mélange gaz d'échappement/air,
la section transversale de sortie (18) du conduit de gaz d'échappement (12) étant disposée sensiblement de manière concentrique dans le conduit d'air (20), et
une orifice circulaire (34) étant disposée en aval de la section transversale de sortie (18) du conduit de gaz d'échappement (12) dans le tunnel de dilution (38),
**caractérisé en ce qu'**en amont de ladite orifice (34) et en aval de la section transversale de sortie (18) du canal de gaz d'échappement (12) des moyens (32, 44, 50) sont disposés qui sont formés par plusieurs tuyaux de sortie (50) disposés symétriquement l'un par rapport à l'autre par rapport à l'axe central, ou sont formés par un corps (44) comprenant une zone sphérique et disposé de manière concentrique dans le tunnel de dilution (38), le flux de gaz d'échappement étant dévié par lesdits moyens (32, 44, 50) de sorte qu'immédiatement en aval des moyens (32, 44, 50), le flux de gaz d'échappement s'écarte de l'axe central du tunnel de dilution (38) de manière axisymétrique.

2. Système de prélèvement d'échantillons de gaz d'échappement à partir de moteurs à combustion interne selon la revendication 1, **caractérisé en ce que** la zone sphérique est un hémisphère (44).

3. Système de prélèvement d'échantillons de gaz d'échappement à partir de moteurs à combustion interne selon l'une quelconque des revendications précédentes, **caractérisé en ce que** la zone sphérique (44) est électropolie.

4. Système de prélèvement d'échantillons de gaz d'échappement à partir de moteurs à combustion interne selon l'une quelconque des revendications précédentes, **caractérisé en ce que** la zone sphérique (44) comprend un trou central (46).

5. Système de prélèvement d'échantillons de gaz d'échappement à partir de moteurs à combustion interne selon l'une quelconque des revendications précédentes, **caractérisé en ce que** la zone sphérique (44) ou la orifice (34) peuvent être chauffées.

6. Système de prélèvement d'échantillons de gaz d'échappement à partir de moteurs à combustion interne selon la revendication 5, **caractérisé en ce qu'**une feuille de chauffage est disposée sur la surface aval de la zone sphérique (44) ou de ladite orifice (34).

7. Système de prélèvement d'échantillons de gaz d'échappement à partir de moteurs à combustion interne selon l'une quelconque des revendications précédentes, **caractérisé en ce que** la surface de projection de la zone sphérique (44) vers l'axe central correspond au moins à la section transversale de sortie (18) du conduit de gaz d'échappement (12).

8. Système de prélèvement d'échantillons de gaz d'échappement à partir de moteurs à combustion interne selon l'une quelconque des revendications précédentes, **caractérisé en ce que** la section transversale d'écoulement de ladite orifice (34) correspond à 1,2 à 1,8 fois la section transversale des moyens (32, 44) pour la déviation d'écoulement.

9. Système de prélèvement d'échantillons de gaz d'échappement à partir de moteurs à combustion interne selon l'une quelconque des revendications précédentes, **caractérisé en ce que** la distance des moyens (32, 44) pour la déviation d'écoulement de la section transversale de sortie (18) du conduit de gaz d'échappement (12) est un tiers jusqu'à un cinquième de la différence de diamètre du conduit d'air (20) et le diamètre du conduit d'échappement (12).

10. Système de prélèvement d'échantillons de gaz d'échappement à partir de moteurs à combustion interne selon l'une quelconque des revendications précédentes, **caractérisé en ce que** la distance entre les moyens (32, 44, 50) pour la déviation du flux et l'orifice (34) est un tiers jusqu'à un cinquième du diamètre du conduit d'air (20).

11. Système de prélèvement d'échantillons de gaz d'échappement à partir de moteurs à combustion interne selon la revendication 1, **caractérisé en ce que** quatre tuyaux de sortie (50) sont disposés décalés de 90° les uns par rapport aux autres.

12. Système de prélèvement d'échantillons de gaz d'échappement à partir de moteurs à combustion interne selon la revendication 11, **caractérisé en ce que** les quatre tuyaux d'échappement (50) s'étendent au voisinage immédiat du conduit de gaz d'échappement (12) ou dans le conduit de gaz d'échappement (12) initialement parallèlement à l'axe central, et dans la suite, hors du conduit d'échappement (12), ils sont courbés s'écartant en continu de l'axe central.

13. Système de prélèvement d'échantillons de gaz d'échappement à partir de moteurs à combustion interne selon la revendication 12, **caractérisée en ce que** les sections transversales de sortie des tuyaux de sortie (50) forment un angle de 30° à 80° avec l'axe central du tunnel de dilution.

14. Système de prélèvement d'échantillons de gaz d'échappement à partir de moteurs à combustion interne selon l'une quelconque des revendications précédentes, **caractérisé en ce que** les moyens (32, 44, 50) pour la déviation du flux sont fixés sur l'orifice (34) par l'intermédiaire d'au moins un, de préférence trois entretoises (48).
